# FASCICULE DE BREVET EUROPEEN

(11) **EP 1 001 818 B1**
(45) Date de publication et mention de la délivrance du brevet: **03.12.2003**
(21) Numéro de dépôt: 98938748.5
(22) Date de dépôt: 15.07.1998
(51) Int. Cl.: A61L 31/00, A61F 2/06, A61K 47/48

(54) **DISPOSITIF IMPLANTABLE RECOUVERT D'UN POLYMERE CAPABLE DE LIBERER DES SUBSTANCES BIOLOGIQUEMENT ACTIVES**
IMPLANTIERBARE VORRICHTUNG MIT EINER POLYMERBESCHICHTUNG ZUR FREISETZUNG VON BIOLOGISCH WIRKSAMEN STOFFEN
IMPLANTABLE DEVICE COATED WITH POLYMER CAPABLE OF RELEASING BIOLOGICALLY ACTIVE SUBSTANCES

(30) Priorité: 16.07.1997 FR 9709001
(43) Date de publication de la demande: 24.05.2000
(73) Titulaire: CENTRE NATIONAL DE LA RECHERCHE SCIENTIFIQUE, 75016 Paris Cédex (FR)
(72) Inventeur: DUBOIS-RANDE, Jean, Luc, F-94000 Créteil (FR); LE DOAN, Trung, F-92160 Antony (FR); PHAM, Minh, Chau, F-92340 Bourg La Reine (FR); PIRO, Benoît, F-17000 La Rochelle (FR); TEIGER, Emmanuel, F-94300 Vincennes (FR); TENU, Jean, Pierre, F-92360 Meudon la Forêt (FR)
(74) Mandataire: L'Helgoualch, Jean
(86) Numéro de dépôt international: FR9801538
(87) Numéro de publication internationale: WO99003517

(56) Documents cités:
- EP-A- 0 623 354
- EP-A- 0 701 802
- EP-A- 0 747 069
- WO-A-97/12899
- DE-C- 4 429 380

## Description

La présente invention concerne un implant recouvert d'un polymère capable de libérer diverses substances, et plus particulièrement un stent recouvert d'un polymère capable d'encapsuler des substances biologiquement actives destinées au traitement de la resténose, et de les libérer localement, ainsi qu'un procédé pour déposer un polymère sur un support.

Dans divers domaines de la médecine, on a mis au point des techniques consistant à introduire des implants ou des prothèses dans le corps humain ou animal afin de corriger ou limiter certaines déficiences.

Ainsi, dans le domaine de la cardiologie, la technique de l'angioplastie par ballonnets des sténoses artérielles est aujourd'hui largement utilisée pour le traitement des lésions vasculaires coronaires ou périphériques responsables de l'angine de poitrine, de l'infarctus du myocarde et de l'artérite des membres inférieurs. Cette technique consiste à introduire, sous contrôle angiographique, une sonde à ballonnet gonflable dans le vaisseau à traiter au niveau de la zone rétrécie, et à rétablir le flux sanguin en écrasant la plaque d'athérome dans la paroi du vaisseau. Il est ainsi généralement possible d'éviter de recourir aux techniques chirurgicales classiques pour parvenir à une revascularisation artérielle ou périphérique.

Cependant, l'angioplastie présente l'inconvénient d'entraîner souvent un nouveau rétrécissement de l'artère, appelé resténose. Dans 30% à 40% des cas environ, cette resténose survient dans un délai de six mois, et elle est imprévisible. Elle est essentiellement due à une cicatrisation rétractile de l'artère et à une prolifération des cellules musculaires lisses de la paroi artérielle, en réponse à l'agression produite par l'introduction du ballonnet. Ces deux phénomènes entraînent un nouveau rétrécissement de la lumière artérielle.

L'administration de médicaments tels que des inhibiteurs de l'enzyme de conversion de l'angiotensine, des anti-inflammatoires, des anti-agrégants plaquettaires, des anticoagulants, etc, n'a pas permis de lutter efficacement contre le phénomène de la resténose.

Pour empêcher la rétraction artérielle, on utilise des prothèses endovasculaires métalliques en forme de petit ressort, appelées stents, que l'on met en place dans l'artère après avoir effectué la dilatation. Le stent a pour effet d'empêcher le rappel élastique immédiat de la paroi de l'artère, et, à plus long terme, de prévenir la constriction de l'artère. Par contre, la présence du stent n'a pas d'effet favorable sur la prolifération des cellules musculaires lisses, et dans certains cas elle peut même l'aggraver.

Il est donc souhaitable de pouvoir associer un traitement médicamenteux efficace à la pose du stent.

On sait que certains oligonucléotides antisens exercent une activité antiproliférative in vitro et in vivo (M.R. Bennett et al. "Inhibition of vascular muscle cell accumulation in vitro and in vivo by c-myc antisense oligonucleotides", J. Clin. Invest. (1994) 93, 820-28). Cependant, aucune technique efficace d'administration de tels composés n'a été décrite. Des dérivés nucléotidiques antisens adsorbés ou encapsulés dans des nanoparticules de polycyanoacrylate d'alkyle sont décrits dans le brevet FR-A-2.724.935 qui propose cette technique pour leur administration intra-tumorale dans le traitement de certains cancers.

Le brevet DE-A-4.429.380 décrit un procédé de préparation de stents à support céramique ou métallique revêtus d'une couche intermédiaire de silicium amorphe et d'une couche de matériau semi-conducteur, ces deux couches étant imbriquées l'une dans l'autre.

Des stents métalliques recouverts d'une première couche composite d'un polymère et d'une substance thérapeutiquement active, revêtue d'une deuxième couche de fibrine, sont décrits dans la demande de brevet EP-A-701.802. Le polymère utilisé est choisi parmi des silicones, des polyuréthannes, des polyesters, des polyéthers et des dérivés vinyliques. D'autre part, selon le brevet EP-A-566.245, la fibrine peut être utilisée dans le traitement de la resténose. Des essais effectués sur des artères coronaires de porc avec divers polymères biodégradables (par exemple acide polyglycolique / acide polylactique, polycaprolactone, etc) ou non biodégradables (polyuréthanne, silicone, téréphtalate de polyéthylène) ont mis en évidence d'importantes réactions inflammatoires accompagnées d'une prolifération fibrocellulaire de la paroi artérielle (W. J. Van der Giessen et al., Circulation, (1996) 94, 1690-97).

Ainsi, il n'existe pas actuellement de système combinant efficacement un stent et une substance antiproliférative des cellules musculaires lisses, pour le traitement de la resténose.

La présente invention a donc pour objet un dispositif implantable dans l'organisme, ou endoprothèse, et en particulier un stent, recouvert d'un polymère susceptible d'encapsuler des substances biologiquement actives de nature anionique ou cationique et de les libérer localement, utilisable dans le traitement de diverses affections, et notamment de la thrombose endoprothétique ainsi que de la resténose dans le domaine de l'angioplastie coronaire et de l'angioplastie des artères périphériques.

L'invention concerne également un procédé permettant de déposer un polymère électroconducteur renfermant une substance biologiquement active, sur un support électroconducteur tel qu'un support métallique, et notamment un stent.

Le procédé conforme à la présente invention permet le dépôt par voie électrochimique d'un polymère électroconducteur, par exemple un polymère dérivé du pyrrole, du naphtalène ou du thiophène, qui peut être obtenu en milieu aqueux dans des conditions douces, c'est-à-dire à température ambiante et dans des conditions salines modérées à partir de monomères disponibles dans le commerce. De plus, le dépôt électrochimique permet un contrôle précis de l'épaisseur de la couche de polymère déposée sur le support, et le milieu utilisé ne contient pas d'oxydant chimique ou de solvant organique qui pourraient présenter des effets nocifs dans l'application envisagée. Dans le cas où la polymérisation électrochimique est difficile ou ne peut pas être envisagée, on peut préparer le polymère en solution dans un solvant approprié, suivant une technique classique, et le déposer sur le support par pulvérisation ou trempage, puis évaporation du solvant.

Le procédé de l'invention comprend essentiellement deux étapes. Dans une première étape, on effectue directement une électropolymérisation sur le support métallique, ou, suivant une variante, on prépare le polymère en solution que l'on dépose sur le support métallique, comme indiqué ci-dessus. Puis, dans une deuxième étape, on effectue une oxydation (si le polymère est cationique) ou une réduction (si le polymère est anionique), et simultanément on fixe la substance biologiquement active sur le polymère. L'oxydation et la réduction, selon les cas, s'effectuent par voie électrochimique, de manière connue en créant une différence de potentiel entre le support électroconducteur et le polymère afin de former des charges positives ou négatives, respectivement, favorisant la fixation de la substance active. Par exemple, la formation d'une matrice polymère favorise la fixation d'une substance active constituée par une base nucléique phosphorylée chargée positivement ; à l'inverse, la formation de charges positives dans le polymère favorise la fixation d'une substance active constituée par exemple par un oligonucléotide ou une protéine portant des sites négatifs comme l'ATP, ou plus généralement toute particule chargée négativement, telle que l'héparine, un vecteur de gènes plasmidiques, ou un fragment d'ADN linéaire ou circulaire de type plasmide.

Suivant une forme préférentielle de mise en oeuvre du procédé de dépôt de polymère électroconducteur conforme à l'invention, la polymérisation par voie électrochimique s'effectue en présence d'un polymère hydrosoluble, choisi parmi un polyéthylène glycol, une polyvinylpyrrolidone, un polyéthylène oxyde, un copolymère d'oxyde d'éthylène et d'oxyde de propylène de type Poloxamer, un acétate de polyéthylène, un polyalcool vinylique, un polyacrylamide, ainsi que des dérivés hydrosolubles du polyuréthanne. La présence d'un tel polymère hydrosoluble dans la matrice polymère permet d'améliorer la perméabilité du polymère aux espèces anioniques, telles que des oligonucléotides, utilisées comme substance active, et facilite la libération contrôlée de ces molécules au contact de solutions contenant des ions compétiteurs, provenant par exemple de l'addition de chlorure de sodium. On utilise de préférence une quantité de polymère représentant entre 4 et 10% de la composition totale.

Plus particulièrement, le film de polymère peut être avantageusement formé en deux étapes, la première consistant à polymériser le monomère à une concentration comprise entre 0,01 et 0,1 M environ, pour former une couche d'accrochage d'épaisseur inférieure à environ 1 µm, la deuxième consistant à prolonger la réaction de polymérisation en présence de polymère hydrosoluble pour obtenir une couche d'épaisseur supérieure à 1 µm, qui peut être comprise entre 2 et 10 µm environ.

Il est avantageux d'utiliser un support métallique réalisé par exemple en acier, en alliage métallique, ou en un métal biocompatible et plus particulièrement en acier inoxydable, tantale, platine, or, alliage nickel-titane ou alliage platine-indium. Un support en acier inoxydable peut être avantageusement utilisé lorsque le milieu électrolytique ne contient pas de chlorure. On peut aussi utiliser un support non métallique tel qu'un polymère chargé électroconducteur biologiquement compatible.

L'oxydation s'effectue, après dépôt du polymère comme indiqué ci-dessus, en appliquant au support un potentiel d'oxydation pendant une période de temps déterminée. Ainsi, par exemple, selon le polymère utilisé, on peut appliquer un potentiel d'environ 0,3 V à 1 V par rapport à une électrode de référence à hydrogène, pendant une période de temps comprise entre 30 minutes et 3 heures environ, en fonction des propriétés recherchées.

Suivant une caractéristique de l'invention, grâce à l'étape d'oxydation, il est possible d'ajuster la capacité de fixation de la substance active, en particulier les oligonucléotides, sur le polymère et d'en contrôler ensuite la libération localement sur le site à traiter, une fois le stent mis en place dans l'organisme. Le chargement de la substance active sur le polymère s'effectue avantageusement simultanément avec l'étape d'oxydation, en ajustant la concentration de substance active dans la solution d'oxydation à une valeur appropriée, qui peut être comprise par exemple entre 1 et 200 µM, selon la substance utilisée et le taux de fixation recherché. Il est ainsi possible de parvenir à un taux de fixation supérieur à plusieurs nanomoles/mg de polymère, associé à une cinétique de libération lente assurant un effet prolongé de la substance active. Cette technique permet d'obtenir par exemple une cinétique de libération de l'ordre de quelques picomoles de substance active par mg de support et par jour.

La cinétique de libération observée en utilisant la technique de l'invention est liée à l'épaisseur du film de polymère. Il est possible, conformément à l'invention, d'ajuster la cinétique de libération de la substance active en choisissant une épaisseur appropriée du polymère sur le support, comprise par exemple entre 2 et 10 µm environ.

La substance active est choisie en fonction de l'utilisation envisagée pour l'implant. Ainsi, dans le cas d'un stent recouvert d'un polymère destiné au traitement de la resténose post-angioplastie, la substance active est de préférence un oligonucléotide antisens capable de bloquer sélectivement l'expression des gènes contrôlant la prolifération des cellules musculaires lisses de la paroi artérielle. On peut utiliser par exemple des oligonucléotides, et plus particulièrement des oligodésoxyribonucléotides (ODN) antisens à structure phosphodiester ou phosphorothioate, dirigés contre le récepteur du facteur de croissance proche de l'insuline (ou IGFR) comme décrit par P. Delafontaine et al. ("Regulation of vascular smooth muscle cell Insuline-like Growth Factor I Receptors by phosphorothioate oligonucleotides" J. Biol. Chem, (1995) 270, 14383-388) et WO-A-96.10401, ou encore des oligo-désoxynucléotides contenant n guanines contiguës, n pouvant varier de 2 à 5 comme décrit par Burgess et al. ("The antiproliferative activity of c-myb and c-myc antisense oligonucleotides in smooth muscle cells is caused by a non-antisens mechanism", Proc. Nat. Acad. Sci. USA (1995) 92, 4051-4055).

Ces oligodésoxyribonucléotides ont généralement une durée de vie très courte à l'état libre dans l'organisme car ils sont rapidement digérés par les nucléases, ce qui limite leur efficacité. La présente invention est donc particulièrement avantageuse car elle permet de protéger ces oligodésoxyribonucléotides dans la matrice de polymère et de les délivrer progressivement sur le site même, où ils peuvent alors agir efficacement.

De plus, conformément à l'invention, l'oligonucléotide peut être marqué radioactivement. L'isotope radioactif permet de contrôler la fixation sur le polymère, et peut aussi induire un effet antiprolifératif sur les cellules musculaires lisses. Le marquage peut être réalisé de manière classique, par exemple au moyen de soufre ³⁵S ou de phosphore ³²P dont la demi-vie est de 87 jours et 15 jours respectivement, et convient à l'évolution dans le temps de la resténose.

Le polymère utilisé pour recouvrir le stent, conformément à la présente invention, est choisi parmi les polymères électroconducteurs présentant des propriétés satisfaisantes de tolérance biologique. On utilise de préférence un polypyrrole, un poly(diaminonaphtalène) ou un poly(3,4-éthylène-dioxythiophène) ou poly(EDT), qui présentent des propriétés de conduction électrique et de résistance mécanique appropriées, sans nuire aux qualités mécaniques du support utilisé.

Il peut être avantageux, conformément à la présente invention, d'appliquer sur le polymère portant l'oligonucléotide, une couche de substance telle que la fibrine, connue pour son activité antithrombotique.

L'invention s'applique tout particulièrement à un stent recouvert d'un polymère contenant une substance active efficace dans le traitement de la resténose. Elle peut s'appliquer plus généralement à tout implant susceptible d'être recouvert d'une couche de polymère cationique (ou cationisable) ou anionique (ou anionisable) pouvant servir de réservoir de substance active portant des charges positives ou négatives, destinée à être libérée localement, par exemple des substances présentant des propriétés pharmacologiques utiles dans le traitement des leucémies, des tumeurs solides et des rejets d'organes.

Les exemples ci-après illustrent l'invention plus en détail sans en limiter la portée.

### Exemple 1

On effectue l'électropolymérisation du 3,4-éthylènedioxythiophène au moyen d'une cellule électrochimique classique à trois électrodes : une contre-électrode de platine, un fil d'argent recouvert de chlorure d'argent servant d'électrode de référence fournissant un potentiel constant, et une électrode de travail de platine pulvérisé sur plaque de verre de 0,6 cm² de surface, donnant un potentiel vis-à-vis de la contre-électrode.

La cuve de la cellule contient la solution de monomère à polymériser et le sel assurant la conduction électrique.

Le monomère est le 3,4-éthylènedioxythiophène auquel on ajoute un polyéthylène glycol (3.10⁻² M) ou une polyvinylpyrrolidone (2.10⁻³ M). La solution électrolytique est du PBS (tampon phosphate salin) à pH 7,4 contenant les sels suivants : KCl (2,7 mM), NaCl (0,14 M), KH₂PO₄ (1,4 mM) et Na₂HPO₄ (6,4 mM).

On ajoute le monomère dans la solution de PBS diluée 15 fois. On utilise la méthode potentiodynamique (voltamétrie cyclique), le potentiel allant de -0,1 V à +1,3 V par rapport à Ag/AgCl pendant 10 à 30 cycles. L'oxydation du monomère commence vers 0,9 V. Le dernier cycle est arrêté au potentiel supérieur pour obtenir un film oxydé homogène. L'épaisseur du film, mesurée au moyen d'un microscope électronique à balayage, est de 2,5 µm (10 cycles) ou 5,4 µm (20 cycles). On peut augmenter l'épaisseur du film en augmentant le nombre de cycles. Cependant, au-delà d'environ 30 cycles, les propriétés mécaniques du film risquent d'être diminuées.

On incorpore ensuite l'un des oligonucléotides indiqués ci-après à titre de substance active, en oxydant le polymère à un potentiel de +0,7 V par rapport à une électrode de référence Ag/AgCl, dans une solution de PBS contenant une quantité d'oligonucléotide égale à 1 µM. Le potentiel est maintenu pendant environ 1h30.

Le film de polymère chargé en oligonucléotide est rincé rapidement à l'eau sur les deux faces de l'électrode de travail.

Les oligodésoxyribonucléotides (ODN) antisens utilisés successivement dans cet exemple sont les suivants :

5'-CTC-TCG-CAC-CCA-TCT-CTC-TCC-TTC-T (phosphorothioate)

IGFR   TCC-GGA-GCC-AGA-CTT-CAT-TC (phosphorothioate)
C-Myc  5'-AAC-GTT-GAG-GGG-CAT (phosphodiester).

Ces ODN sont purifiés par chromatographie liquide de haute performance (HPLC) sur colonne de phase inverse C18 en utilisant des tampons d'élution acétate de tétraéthylammonium / acétonitrile.

Afin de contrôler l'encapsulation des oligodésoxyribonucléotides (ODN) dans le film de polymère, on procède par marquage radioactif suivant une technique classique, en utilisant le ³²P comme marqueur isotopique.

Le marquage s'effectue par transfert d'un groupe phosphate radioactif ³²P d'ATP vers la position 5' de l'ODN, par la polynucléotide kinase à 37°C en milieu tampon acétate.

### Exemple 2

On effectue l'électropolymérisation d'un dérivé du naphtalène, la 5-amino-1,4-naphtoquinone (ANQ, concentration 10⁻²M), dans une solution d'acétonitrile contenant du LiClO₄ 10⁻¹M. On utilise la méthode potentiodynamique (voltamétrie cyclique) en faisant varier le potentiel de 0,5 V à 1,45 V par rapport à une électrode de référence au calomel (ECS) pendant 40 minutes.

L'épaisseur du film, mesurée au moyen d'un microscope électronique à balayage, est de 1 µm.

La substance active chargée positivement est ensuite incorporée en réduisant le polymère à un potentiel de -0,3 V par rapport à une ECS dans une solution aqueuse de pH environ 7 contenant la substance chargée positivement. Le potentiel est maintenu pendant environ 20 minutes.

### Exemple 3

On procède comme dans l'Exemple 1 en utilisant comme monomère le 3,4-éthylènedioxythiophène, mais en formant le film de polymère en deux étapes successives.

Dans une première étape, on polymérise le monomère dans le même milieu PBS que dans l'Exemple 1, dilué 15 fois. La concentration de monomère est de 3.10⁻² M. La technique utilisée est identique à celle de l'Exemple 1, mais en effectuant seulement 2 cycles de balayage, de manière à former un film d'environ 0,5 µm d'épaisseur, constituant une couche d'accrochage.

Dans une deuxième étape, on ajoute au milieu une polyvinylpyrrolidone de masse molaire 40.000 environ, à la concentration de 2.10⁻³ M, et on prolonge la polymérisation jusqu'à obtenir un film de poly(3,4-éthylènedioxythiophène) présentant une épaisseur de 6 µm environ.

Les essais effectués en utilisant un stent de platine comme support, ont mis en évidence les excellentes propriétés mécaniques du revêtement polymère qui possède une bonne adhérence.

### Exemple 4

Les expérimentations effectuées avec les stents de l'invention ont mis en évidence une très bonne tolérance après implantation in vivo, permettant d'envisager leur utilisation dans le traitement de la resténose.

Ainsi, un stent de platine à structure maillée, recouvert de polymère, préparé comme indiqué dans l'Exemple 1, a été implanté dans l'aorte abdominale de deux lapins New-Zealand pesant 3,5 kg. Un stent identique, mais non recouvert de polymère, a été implanté chez le même animal, dans la même aorte mais 2 cm en amont et sert de stent de contrôle. Un traitement par l'aspirine a été commencé un jour avant l'intervention (0,07 mg/ml d'eau de boisson), puis prolongé pendant toute la durée de l'implantation. Les deux artères ont été prélevées, trois et quinze jours après la mise en place du stent, et examinées en histologie standard après fixation.

Au 3ème jour, on n'observe pas de thrombus pariétal. On note une prolifération modérée, ne recouvrant pas les mailles du stent, de cellules musculaires lisses, au niveau des zones de contact entre le stent et la paroi artérielle. On note la présence de quelques cellules circulantes, hématies et polynucléaires, ainsi que de quelques fibres de collagène. L'aspect est identique au niveau des deux stents, c'est-à-dire le stent de contrôle et le stent recouvert de polymère.

Au 15ème jour, aucun thrombus pariétal n'est observé. La prolifération des cellules musculaires lisses est un peu plus accentuée et est constituée des mêmes éléments que ci-dessus. La couche proliférative est plus organisée, recouvre les mailles du stent recouvert de polymère et présente une couche de cellules endothéliales en surface, favorisant ainsi la stabilisation du processus.

Ces essais confirment les propriétés de bio- et hémocompatibilité du polymère poly(EDT) utilisé conformément à l'invention.

## Revendications

1. Dispositif implantable dans l'organisme **caractérisé en ce qu'**il comprend un support électroconducteur recouvert d'une couche de polymère électroconducteur sur/dans laquelle est encapsulée au moins une substance biologiquement active de nature anionique ou cationique.

2. Dispositif selon la revendication 1, **caractérisé en ce que** le polymère est un polymère dérivé du pyrrole, du naphtalène ou du thiophène.

3. Dispositif selon la revendication 2, **caractérisé en ce que** le polymère est un poly(3,4-éthylène-dioxythiophène).

4. Dispositif selon l'une quelconque des revendications précédentes, **caractérisé en ce que** la substance biologiquement active est une base nucléique phosphorylée, un oligonucléotide antisens, ou un vecteur de gènes plasmidiques.

5. Dispositif selon la revendication 4, **caractérisé en ce que** la substance biologiquement active est un oligodésoxyribonucléotide (ODN) antisens à structure phosphodiester ou phosphorothioate.

6. Dispositif selon l'une quelconque des revendications précédentes, **caractérisé en ce que** le support électroconducteur est un métal choisi parmi un acier inoxydable, le tantale, le platine, l'or, un alliage nickel-titane ou un alliage platine-indium.

7. Dispositif selon la revendication 1, **caractérisé en ce qu'**il est constitué par un stent.

8. Procédé de dépôt d'un polymère sur un support électroconducteur pour dispositif implantable dans l'organisme selon la revendication 1, **caractérisé en ce que**, dans une première étape, on effectue directement une électropolymérisation sur le support électroconducteur, ou, suivant une variante, on prépare le polymère en solution que l'on dépose sur le support électroconducteur, puis, dans une deuxième étape, on effectue une oxydation, ou une réduction, et on fixe la substance biologiquement active de nature anionique, ou cationique, sur le polymère.

9. Procédé selon la revendication 8, **caractérisé en ce que** l'électropolymérisation s'effectue en présence d'un polymère hydrosoluble.

10. Procédé selon la revendication 9, **caractérisé en ce que** le polymère hydrosoluble est choisi parmi un polyéthylène glycol, une polyvinylpyrrolidone, un polyéthylène oxyde, un copolymère d'oxyde d'éthylène et d'oxyde de propylène de type Poloxamer, un acétate de polyéthylène, un polyalcool vinylique, un polyacrylamide, ainsi que des dérivés hydrosolubles du polyuréthane.

11. Procédé selon la revendication 8, **caractérisé en ce que** l'oxydation est effectuée par voie électrochimique.

12. Procédé selon l'une quelconque des revendications 8 à 11, **caractérisé en ce que** le chargement de la substance active sur le polymère est effectué simultanément avec l'étape d'oxydation ou de réduction.

## Patentansprüche

1. In den Organismus implantierbare Vorrichtung, **dadurch gekennzeichnet, dass** sie einen elektrisch leitenden Träger umfasst, der mit einer Schicht aus elektrisch leitendem Polymer beschichtet ist, auf/in der zumindest eine anionische oder kationische biologisch aktive Substanz eingekapselt ist.

2. Vorrichtung nach Anspruch 1, **dadurch gekennzeichnet, dass** das Polymer ein von Pyrol, Naphthalin oder Thiophen abgeleitetes Polymer ist.

3. Vorrichtung nach Anspruch 2, **dadurch gekennzeichnet, dass** das Polymer ein Poly(3,4-ethylendioxythiophen) ist.

4. Vorrichtung nach einem der vorangegangenen Ansprüche, **dadurch gekennzeichnet, dass** die biologisch aktive Substanz eine phosphorylierte Nucleinsäurebase, ein Antisense-Oligonucleotid oder ein Plasmid-Gen-Vektor ist.

5. Vorrichtung nach Anspruch 4, **dadurch gekennzeichnet, dass** die biologisch aktive Substanz ein Antisense-Oligodesoxyribonucleotid (ODN) mit Phosphodiester- oder Thiophosphatstruktur ist.

6. Vorrichtung nach einem der vorangegangenen Ansprüche, **dadurch gekennzeichnet, dass** der elektrisch leitende Träger ein aus Edelstahl, Tantal, Platin, Gold, einer Nickel-Titan-Legierung oder einer Platin-Indium-Legierung ausgewähltes Metall ist.

7. Vorrichtung nach Anspruch 1, **dadurch gekennzeichnet, dass** sie aus einem Stent besteht.

8. Verfahren zur Ablagerung eines Polymers auf einem elektrisch leitenden Träger als in den Organismus implantierbare Vorrichtung gemäß Anspruch 1, **dadurch gekennzeichnet, dass** in einem ersten Schritt direkt auf dem elektrisch leitenden Träger eine Elektropolymerisation durchgeführt wird oder gemäß einer Ausführungsform das Polymer in einer Lösung hergestellt wird, die auf dem elektrisch leitenden Träger aufgetragen wird, wonach in einem zweiten Schritt eine Oxidation oder eine Reduktion durchgeführt wird und die anionische oder kationische biologisch aktive Substanz auf dem Polymer fixiert wird.

9. Verfahren nach Anspruch 8, **dadurch gekennzeichnet, dass** die Elektropolymerisation in Gegenwart eines wasserlöslichen Polymers durchgeführt wird.

10. Verfahren nach Anspruch 9, **dadurch gekennzeichnet, dass** das wasserlösliche Polymer aus einem Polyethylenglykol, einem Polyvinylpyrrolidon, einem Polyethylenoxid, einem Ethylenoxid-Proyplenoxid-Copolymer vom Polyoxamer-Typ, einem Polyethylenacetat, einem Polyvinylalkohol, einem Polyacrylamid sowie hydrolysierbaren Polyurethanderivaten ausgewählt ist.

11. Verfahren nach Anspruch 8, **dadurch gekennzeichnet, dass** die Oxidation auf elektrochemischem Weg erfolgt.

12. Verfahren nach einem der Ansprüche 8 bis 11, **dadurch gekennzeichnet, dass** das Beladen des Polymers mit der aktiven Substanz gleichzeitig mit dem Oxidations- oder Reduktionsschritt durchgeführt wird.

## Claims

1. Device which can be implanted in the body, **characterized in that** it comprises an electrically conducting support covered with a layer of electrically conducting polymer, on/in which layer is encapsulated at least one biologically active substance with an anionic or cationic nature.

2. Device according to Claim 1, **characterized in that** the polymer is a polymer derived from pyrrole, naphthalene or thiophene.

3. Device according to claim 2, **characterized in that** the polymer is a poly(3,4-ethylenedioxythiophene).

4. Device according to any one of the preceding claims, **characterized in that** the biologically active substance is a phosphorylated nucleic base, an antisense oligonucleotide or a vector for plasmid genes.

5. Device according to Claim 4, **characterized in that** the biologically active substance is an antisense oligodeoxyribonucleotide (ODN) comprising a phospho-diester or phosphorothioate structure.

6. Device according to any one of the preceding claims, **characterized in that** the electrically conducting support is a metal selected from a stainless steel, tantalum, platinum, gold, a nickel-titanium alloy or a platinum-indium alloy.

7. Device according to Claim 1, **characterized in that** it is composed of a stent.

8. Process for the deposition of a polymer on an electrically conducting support for a device which can be implanted in the body according to Claim 1, **characterized in that**, in a first stage, an electropolymerization is carried out directly on the electrically conducting support or, according to an alternative form, the polymer is prepared in solution and is deposited on the electrically conducting support and then, in a second stage, an oxidation or a reduction is carried out and the biologically active substance with an anionic or cationic nature is attached to the polymer.

9. Process according to Claim 8, **characterized in that** the electropolymerization is carried out in the presence of a water-soluble polymer.

10. Process according to Claim 9, **characterized in that** the water-soluble polymer is selected from a polyethylene glycol, a polyvinylpyrrolidone, a polyethylene oxide, a copolymer of ethylene oxide and of propylene oxide of Poloxamer type, a polyethylene acetate, a poly(vinyl alcohol), a polyacrylamide and water-soluble polyurethane derivatives.

11. Process according to Claim 8, **characterized in that** the oxidation is carried out electrochemically.

12. Process according to any one of Claims 8 to 11, **characterized in that** the charging of the active substance to the polymer is carried out simultaneously with the oxidation or reduction stage.
